Europäisches Patentamt

European Patent Office

Office européen des brevets

(19)

(11) Veröffentlichungsnummer: **0 159 510**
**B1**

(12) **EUROPÄISCHE PATENTSCHRIFT**

(45) Veröffentlichungstag der Patentschrift: 30.05.90

(51) Int. Cl.⁵: **A 61 F 2/32**

(21) Anmeldenummer: **85102694.8**

(22) Anmeldetag: **09.03.85**

(60) Teilanmeldung **89111097.5 eingereicht am 09/03/85.**

(54) Hüftgelenkendoprothese.

(30) Priorität: **14.04.84 DE 8411765 u**

(43) Veröffentlichungstag der Anmeldung:
**30.10.85 Patentblatt 85/44**

(45) Bekanntmachung des Hinweises auf die
Patenterteilung:
**30.05.90 Patentblatt 90/22**

(84) Benannte Vertragsstaaten:
**DE**

(56) Entgegenhaltungen:
**DE-A-3 002 443**
**DE-C- 837 294**
**DE-U-8 127 991**
**DE-U-8 212 689**
**FR-A-2 194 123**
**FR-A-2 528 307**
**US-A-2 719 522**
**US-A-4 406 023**

(73) Patentinhaber: **HOWMEDICA INTERNATIONAL, INC. Zweigniederlassung Kiel**
**Professor-Küntscher-Strasse 1-5**
**D-2314 Schönkirchen (DE)**

(72) Erfinder: **Beck, Heinrich, Prof. Dr.**
**Rudelsweier 12 1/2**
**D-8520 Erlangen (DE)**
Erfinder: **Richter, Karl Manfred, Dipl.-Ing.**
**Haferkamp 14**
**D-2304 Wendtorf (DE)**

(74) Vertreter: **Dipl.-Ing. H. Hauck Dipl.-Phys. W. Schmitz Dipl.-Ing. E. Graalfs Dipl.-Ing. W. Wehnert Dr.-Ing. W. Döring**
**Neuer Wall 41**
**D-2000 Hamburg 36 (DE)**

Courier Press, Leamington Spa, England.

EP 0 159 510 B1

**Beschreibung**

Die Erfindung bezieht sich auf eine Hüftgelenkendoprothese nach dem Oberbegriff des Patentanspruchs 1.

Aus der DE-U-82 12 689 ist ein Prothesen-Schaft bekannt geworden, der über den größten Teil seiner Länge vom distalen Ende her gesehen gerade und mit gleichmäßigem kreisförmigem Querschnittsprofil versehen ist. Seine Außenfläche ist mit kugeligen Erhebungen versehen, um ein besseres Einwachsen im Knochenkanal zu gewährleisten. Der Schaft wird ohne Knochenzement im Knochenkanal verankert. Da er indessen ein kreisrundes Profil hat, ist die Drehsicherung nicht besonders ausgeprägt. Auch ist das Trägheitsmoment des kreisrunden Querschnitts nicht so hoch, so daß u.U. zu große Durchbiegungen auftreten.

Aus der FR-A-25 28 307 ist eine femorale Endoprothese bekannt geworden, deren gekrümmter Schaft im Querschnitt quadratisch ist, wobei in jeweils einer Fläche eine Nut eingeformt ist. Ein derartiger bananenförmig gekrümmter Schaft ist üblicherweise nur mit Zement implantierbar. Außerdem ist sein Kragen so ausgeformt, daß er mit Hilfe von Knochenschrauben fixiert werden kann. Es versteht sich, daß die Rotationsstabilität durch Knochenschrauben verbessert wird. Mit Knochenzement implantierte Schäfte haben jedoch den großen Nachteil, daß sie bei einer Reoperation außerordentlich schwer zu extrahieren sind.

Aus der FR-A-21 94 123 ist ein gattungsgemäßer Prothesenschaft mit annähernd quadratischem Querschnitt bekannt geworden, der einen relativ schlanken geraden distalen Abschnitt aufweist und dessen Oberfläche durch eine Vielzahl noppenartiger Erhebungen stark aufgerauht ist. Ein derartiger Schaft ist zwar zementlos implantierbar, jedoch wegen der ausgeprägt rauhen Oberfläche schwer in den Knochenkanal einzutreiben.

Ausgehend von einer derartige Prothese ist es daher die Aufgabe der Erfindung, eine zementlos implantierbare Hüftgelenkendoprothese zu schaffen, die leicht in den Knochenkanal eingeschlagen werden kann, jedoch einen wirksamen Sitz im Knochenkanal gewährleistet und die zugleich ein hohes Trägheitsmoment bezüglich aller Achsen aufweist.

Diese Aufgabe wird durch die Merkmale des Kennzeichnungsteils des Patentanspruchs 1 gelöst.

Die erfindungsgemäße Endoprothese weist einen Schaft auf, der in der Anterior-Posterior-Ebene nach distal schwach konisch zuläuft. Auf der lateralen bzw. medialen Seite sind achsparallele eng nebeneinander liegende ein Zahnprofil ergebende Nuten geformt, die ein leichtes Einschlagen in den Knochenkanal erlauben, jedoch eine hohe Rotationsstabilität gewährleisten durch einen sicheren Griff im Knochenkanal. Die Noppen auf der Anterior- und Posterior-Seite sind mit Abständen zueinander angeordnet, die etwas geringer als ihre Abmessungen sind. Sie ermöglichen ein Einwachsen von Knochensubstanz.

Es hat sich gezeigt, daß ein Prothesenschaft mit den erfindungsgemäßen konstruktiven Merkmalen besonders wirksam und kraftschlüssig im Femurkanal verankert werden kann. Der quadratische Querschnitt ermöglicht bezüglich aller Achsen ein relativ hohes Trägheitsmoment und sichert neben den zahnförmigen Nuten den Schaft gegen Verdrehung. Wie bereits erwähnt, ermöglichen die Noppen ein "Hineinwachsen" von Gewebe, gleichwohl sind sie nicht bei dem Einschlagen hinderlich. Der Durchmesser liegt vorzugsweise bei 2 mm mit einer Höhe von 1, 2mm.

Der Schaft und/oder eine möglicherweise vorgesehene prothetische Pfanne werden nach einer Ausgestaltung der Erfindung aus einer Eisen und Aluminium enthaltenden Titanlegierung geformt. Die Legierung lautet vorzugsweise $TiAl_5Fe_{2,5}$. Eine derartige Legierung ist außerordentlich gewebeverträglich und besitzt zudem einen Elastizitätsmodul, der erheblich kleiner ist als bei den herkömmlichen Legierungen. Im Fall der bevorzugten Ausführungsform ist der E-Modul lediglich halb so groß. Dadurch nähert sich die Elastizität z.B. des Prothesenschaftes der des Knochens an, so daß das Ausmaß von Relativbewegungen zwischen Knochen und Prothese bei einer dynamischen Belastung reduziert wird. Dadurch ist auch die Belastung der Prothese im Hinblick auf ihre Lockerung der Verankerung im Knochen verringert.

Der flügelartige Ansatz lateral am proximalen Teil des Schaftes nach einer Ausgestaltung der Erfindung verhindert ebenfalls eine Rotation des Schaftes. Er ist vorzugsweise mit einer langgestreckten Öffnung versehen, über die im Fall einer Reoperation der femorale Endoprothesenteil extrahiert werden kann. Die Öffnung ermöglicht jedoch auch ein Hindurchwachsen von Gewebe und damit eine verbesserte Verankerung des Schaftes im Knochen.

Die Erfindung wird nachfolgend anhand von Zeichnungen näher erläutert.

Fig. 1 zeigt die Vorderansicht des femoralen Teils einer Hüftgelenkendoprothese nach der Erfindung.

Fig. 2 zeigt die laterale bzw. mediale Ansicht des Schaftes des femoralen Teils nach Fig. 1.

Fig. 3 zeigt einen Schnitt durch die Prothese nach Fig. 1 entlang der Linie 3-3.

Fig. 4 zeigt einen Schnitt durch die Prothese nach Fig. 1 entlang der Linie 4-4.

Fig. 5 zeigt einen Schnitt durch die Prothese nach Fig. 1 entlang der Linie 5-5.

Fig. 6 zeigt einen Schnitt durch die Prothese nach Fig. 1 entlang der Linie 6-6.

Fig. 7 zeigt die vergrößerte Einzelheit 7 nach Fig. 5.

Fig. 8 zeigt halb im Schnitt, halb in Seitenansicht den äußeren Teil einer Hüftkappe, wie sie in Verbindung mit dem femoralen Teil der Hüftgelenkendoprothese eingesetzt werden kann.

Fig. 9 zeigt eine Oberansicht des Teils nach Fig. 8.

Fig. 10 zeigt die Einzelheit 10 der Darstellung nach Fig. 8 in vergrößerter Form.

Fig. 11 zeigt teils im Schnitt, teils in Seitenansicht den inneren Teil der Hüftkappe.

Bevor auf die in den Zeichnungen dargestellten Einzelheiten näher eingegangen wird, sei vorangestellt, daß jedes der beschriebenen Teile für sich oder in Verbindung mit Merkmalen der Ansprüche von erfindungswesentlicher Bedeutung ist.

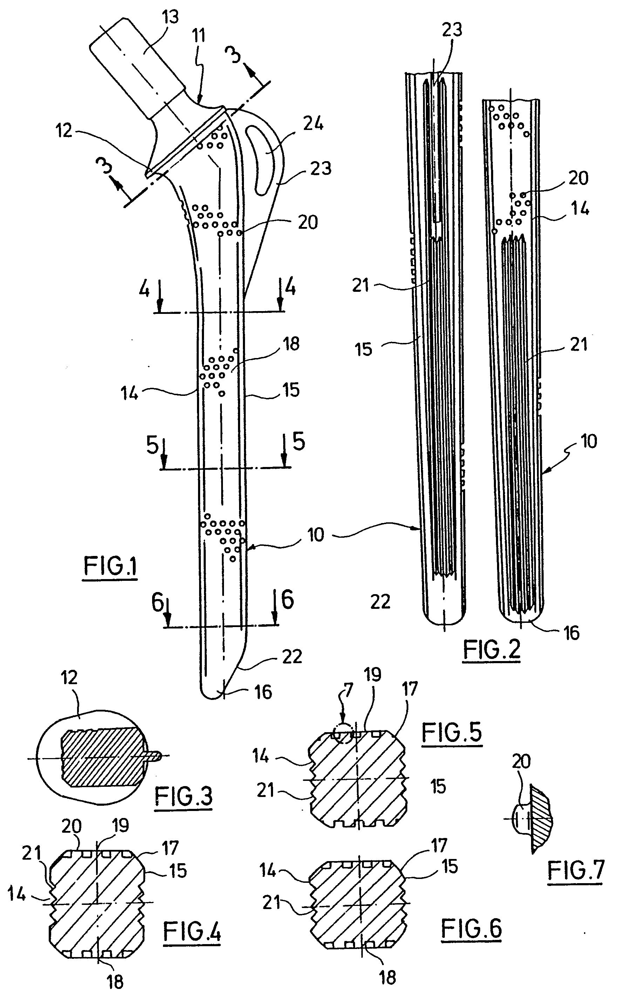

Der in Fig. 1 gezeigte femorale Prothesenteil besitzt einen Schaft 10 und einen Hals 11. Zwischen Schaft 10 und Hals 11 ist ein radial umlaufender Kragen 12 geformt, der mit gerundetem Übergang in den Hals 11 übergeht. An der Unterseite ist er flach ausgebildet. An den Hals 11 schließt sich ein Konus 13 an, auf den eine mit einer Konusbohrung versehene Gelenkkugel aufgesetzt wird.

Der dargestellte femorale Teil besteht vorzugsweise aus einer Eisen und Aluminium enthaltenden Titanlegierung, etwa $TiAl_5Fe_{2,5}$.

Ausgehend vom distalen Ende verlaufen die mediale Seite 14 und die laterale Seite 15 des Schaftes 10 annähernd parallel zueinander, d.h. der Schaft hat in dieser Ebene annähernd gleiche Breite. Die laterale Seite 15 bewegt sich zum distalen Ende hin achseinwärts, so daß eine einseitig angeschärfte Spitze 16 gebildet ist. Nach oben verbreitert sich der Schaft in der beschriebenen Ebene keilförmig bis zum Kragen 12. Gleichzeitig geht er in einer schwachen Biegung in die Achsrichtung des Konus 13 über.

Wie aus Fig. 2 zu erkennen, ist der Schaft 10 in der Anterior-Posterior-Ebene schwach konisch geformt mit einer Verbreiterung nach proximal. Aus den Figuren 4 bis 5 ist zu erkennen, daß der Schaft 10 über den größten Teil seiner Länge annähernd quadratischen Querschnitt aufweist mit stark abgerundeten Längskanten, wie etwa bei 17 dargestellt. Vorderseite 18 und Rückseite 19 des Schaftes 10 sind mit Noppen 20 versehen, von denen einer vergrößert in Fig. 7 dargestellt ist. Die Noppen haben zum Beispiel eine Höhe von 1,2 mm, einen Durchmesser von 2 mm und einen Abstand voneinander von 1,75 mm. Diese Werte sind jedoch beispielhaft, geben indessen die Relation zueinander wieder. Die im Querschnitt kreisförmigen Noppen sind an den Kanten und Übergängen stark gerundet, um scharfe Kanten zu vermeiden.

Medial und lateral ist der Schaft 10 mit einer Reihe paralleler, im Schnitt dreieckförmiger Nuten 21 versehen. Die Nuten liegen so dicht beieinander, daß sich ein gleichmäßiges Zahnprofil ergibt mit im Querschnitt dreieckförmigen Zähnen. Auf der lateralen Seite 15 verlaufen die Nuten bis zur Schrägfläche 22, während sie auf der medialen Seite 14 bis annähernd zur Spitze 16 verlaufen. Sie enden jedoch nach drei Viertel der Höhe von distal gesehen. Oberhalb der Nuten 21 befinden sich ebenfalls Noppen 20.

An der lateralen Seite 15 ist unterhalb des Kraftens 12 ein flossenartiger oder flügelartiger Ansatz 23 angeformt, der sich in distaler Richtung dreieckförmig in Richtung des Schaftes 10 verjüngt und im proximalen Bereich in einem Bogen in den proximalen Abschnitt des Schaftes 10 übergeht. Er ist mit einer langgestreckten ebenfalls gebogenen Öffnung 24 versehen. Jeweils eine Nut 21 erstreckt sich beidseitig des Ansatzes 23 nach oben, wie aus der linken Darstellung nach Fig. 2 hervorgeht. Die Enden der Nuten sind schräg ansteigend geformt. Auf jeder Seite 14, 15 des Schaftes 10 sind vier Nuten geformt. Ihre Breite überdeckt annähernd zwei Drittel der Flächenbreite.

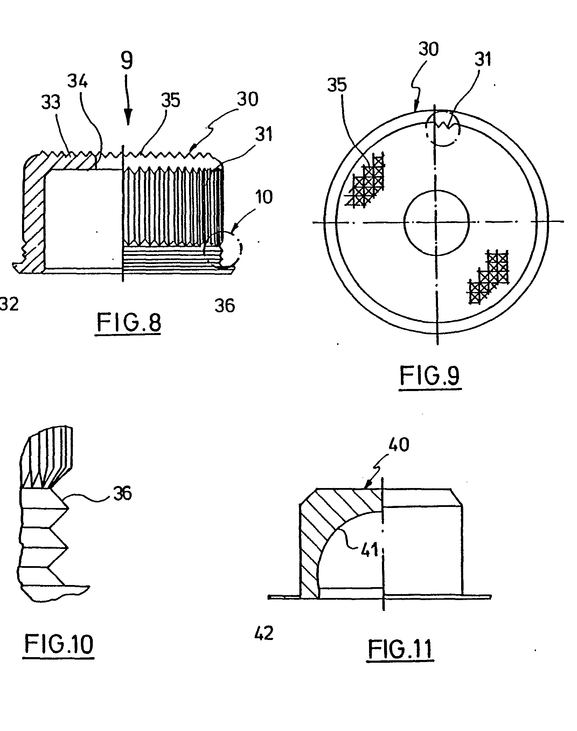

Die Figuren 8 bis 10 zeigen eine mit 30 bezeichnete Hüftpfanne, wie sie in Verbindung mit dem femoralen Teil verwendet werden kann. Sie besteht vorzugsweise aus dem gleichen Material wie der Schaft. Im zylindrischen Mantel ist eine Reihe von achsparallelen Nuten 31 geformt, die im Querschnitt dreieckförmig sind und so aneinanderliegen, daß dazwischen dreieckförmige Zähne gebildet sind. Der Mantel ist an der offenen Seite mit einem radial nach außen weisenden Flansch 32 versehen. Auf der gegenüberliegenden Seite ist ein Boden 33 geformt mit einer mittigen Öffnung 34. Auf der Außenseite des Bodens 33 ist ein Muster von pyramidenartigen Erhebungen 35 geformt.

Die Nuten 31 enden vor dem Eintrittsende der äußeren Schale 30. Dazwischen sind drei Umfangsnuten 36 geformt, die zwischen sich dreieckförmige Rippen oder Zähne bilden. Die Tiefe der Nuten 36 entspricht der Tiefe der Nuten 31, wie aus Fig. 10 zu erkennen. Die den Längsnuten 31 benachbarte Umfangsnut 36 ist so gelegt, daß die eine Nutflanke von den Enden der Zähne zwischen den Längsnuten 31 gebildet wird (siehe ebenfalls Fig. 10). Die äußere Schale 30 wird ohne Knochenzement in eine vorgebohrte Öffnung des Beckenknochens eingesetzt. Die Längsnuten 31 verhindern die Rotation, und die Umfangsnuten 36 wirken der Extraktion entgegen.

Der zylindrische Innenraum der äußeren Schale 30 nimmt eine Innenschale 40 aus Polyethylen auf. Die Innenschale 40 ist mit den Außenabmessungen so gewählt, daß sie passend in den Innenraum der äußeren Schale 30 einsetzbar ist. Die Innenschale 40 weist eine kugelige Höhlung 41 auf zur passenden und schnappenden Aufnahme der nicht gezeigten Gelenkkugel. Die Innenschale 40 besitzt ebenfalls an der offenen Seite einen radial nach außen weisenden Flansch 42, der sich von unten gegen die Stirnseite und den Flansch 32 der äußeren Schale 30 anlegt. Der Flansch 42, der verhältnismäßig dünn ausgeführt ist, verhindert, daß die Gelenkkugel beim Einsetzen gegen metallisches Material stößt.

Die gleichmäßig auf den einzelnen Flächen verteilten Noppen 20 können im Gießverfahren hergestellt oder mechanisch nach dem Gießen eines Rohlings geformt werden.

Die Ausbildung des Schaftes 10, der besonders für Reoperationen geeignet ist, weist ein großes Volumen auf bei günstiger Geometrie zur Herstellung der Noppen. An der lateralen, Seite 15 sind Längsnuten besser als Noppen, da sie die höchstbeanspruchte Seite darstellt.

## Patentansprüche

1. Hüftgelenkendoprothese mit einem femoralen Teil, der einen aus einer Metallegierung geformten Schaft (10) und einen Gelenkkopf aufweist, wobei der Gelenkkopf mit der natürlichen oder einer prothetischen, ebenfalls aus einer Metallegierung bestehenden Hüftpfanne zusammenwirkt, wobei der Schaft (10) in der Lateral-Medial-Ebene wenigstens im Mittenbereich annähernd die gleiche Breite aufweist, der Querschnitt des Schaftes (10) annähernd quadratisch ist und auf der Posterior- und der Anterior-Seite (19, 18) des Schaftes (10) Noppen (20) geformt sind, dadurch gekennzeichnet, daß der Schaft (10) gerundete Längskanten (17) aufweist und in der Anterior-Posterior-Ebene zum distalen Ende hin schwach konisch zuläuft, daß auf der lateralen bzw. medialen Seite (15, 14) des Schaftes (10) achsparallele, eng nebeneinander liegende, ein Zahnprofil ergebende Nuten (21) geformt sind, und daß bei den Noppen (20) deren Abstand voneinander etwas geringer ist als deren Abmessungen.

2. Prothese nach Anspruch 1, dadurch gekennzeichnet, daß die Noppen (20) im Querschnitt kreisförmig sind und eine Höhe aufweisen, die etwas geringer als der Durchmesser ist.

3. Prothese nach Anspruch 1 oder 2, dadurch gekennzeichnet, daß der Schaft (10) und/oder die prothetische Hüftpfanne (30) aus einer Eisen und Aluminium enthaltenden Metalllegierung gußgeformt ist.

4. Prothese nach Anspruch 3, dadurch gekennzeichnet, daß die Legierung $TiAl_5Fe_{2,5}$ lautet.

5. Prothese nach einem der Ansprüche 1 bis 4, dadurch gekennzeichnet, daß lateral am proximalen Teil (14) des Schaftes (10) ein flügelartiger Ansatz (23) angeformt ist.

6. Prothese nach Anspruch 5, dadurch gekennzeichnet, daß der Ansatz (23) eine langgestreckte Öffnung (24) aufweist.

7. Prothese nach einem der Ansprüche 1 bis 6, dadurch gekennzeichnet, daß der Winkel zwischen der Schaftachse und dem Kragen (12) etwa 55° beträgt.

8. Prothese nach einem der Ansprüche 1 bis 7, dadurch gekennzeichnet, daß die Nuten (21) auf der medialen Seite (14) des Schaftes (10) sich vom distalen Ende aus nur bis 3/4 der Höhe des Schaftes (10) erstrecken und oberhalb der Nuten (21) Noppen (20) geformt sind.

## Revendications

1. Endoprothèse d'articulation de la hanche comportant une partie fémorale, qui présente une tige (10) en alliage métallique et une tête d'articulation, la tête d'articulation coopérant avec la cavité naturelle d'articulation de la hanche ou avec une cavité prothétique d'articulation de la hanche également en alliage métallique, la tige (10) présentant dans le plan médial-latéral, au moins dans la zone centrale, une largeur à peu près constante, la coupe transversale de la tige (10) étant à peu près carrée et des saillants (20) étant formés sur le côté postérieur et le côté antérieur (19, 18) de la tige (10), caractérisée en ce que la tige (10) présente des arêtes longitudinales arrondies (17) et a, dans le plan antéro-postérieur, un tracé légèrement conique vers l'extrémité distale, en ce que des encoches (21) produisant un profil denté, parallèles à l'axe et situées proches les unes des autres sont formées sur le côté latéral ou médial (15, 14) de la tige (10), et en ce que la distance de ces encoches entre elles, au voisinage des saillants (20), est un peu plus faible que leurs dimensions.

2. Prothèse selon la revendication 1, caractérisée en ce que les saillants (20) sont de coupe transversale circulaire et d'une hauteur qui est un peu plus petite que leur diamètre.

3. Prothèse selon la revendication 1 ou 2, caractérisée en ce que la tige (10) et/ou la cavité prothétique d'articulation de hanche (30) sont formées par coulée d'un alliage métallique comprenant du fer et de l'aluminium.

4. Prothèse selon la revendication 5, caractérisée en ce que l'alliage est $TiAl_5Fe_{2,5}$.

5. Prothèse selon l'une des revendications 1 à 4, caractérisée en ce qu'un appendice en forme d'ailette (23) est formé latéralement sur la partie proximale de la tige (10).

6. Prothèse selon la revendication 5, caractérisée en ce que l'appendice (23) comporte un orifice allongé (24).

7. Prothèse selon l'une des revendications 1 à 6, caractérisée en ce que l'angle entre l'axe de tige et le col (12) est d'environ 55°.

8. Prothèse selon l'une des revendications 1 à 7, caractérisée en ce que les encoches (21) sur le côté médial (14) de la tige (10) ne s'étendent que jusqu'aux 3/4 de la hauteur de la tige (10) depuis l'extrémité distale et en ce que des saillants (20) sont formés au-dessus des encoches (21).

## Claims

1. A hip joint endo-prosthesis with a femoral member comprising a shaft (10) formed of a metal alloy and a joint head, the joint head cooperating with the natural or a prosthetic hip cup also formed of a metal alloy, the shaft (10) in the lateral medial plane thereof has at least in the middle area thereof the same width, the cross section of the shaft (10) being approximately square and burls (20) being formed at the posterior and anterior sides (18, 19) of the shaft (10), characterized in that the shaft (10) comprises rounded longitudinal edges (17) and extends slightly conical in the anterior-posterior plane towards the distal end, that grooves (21) are positioned parallel with the axis in close distance side-by-side at the lateral or medial side (14, 15), respectively, of the shaft (10), the grooves defining a tooth profile, and that the distances between the burls (20) are slightly smaller than the dimensions thereof.

2. Prosthesis as claimed in claim 1, characterized in that the burls (20) comprise a circular cross section and a height being somewhat smaller than the diameter thereof.

3. Prosthesis as claimed in claim 1 or 2, characterized in that the shaft (10) and/or the prosthetic hip cup (30) are cast of a metal alloy containing iron and aluminum.

4. Prosthesis as claimed in claim 3, characterized in that the alloy is $TiAl_5Fe_{2,5}$.

5. Prosthesis as claimed in one of the claims 1 to 4, characterized in that a wing-like extension (23) is formed at the proximal part (14) of the shaft (10).

6. Prosthesis as claimed in claim 5, characterized in that the extension (23) comprises an elongated aperture (24).

7. Prosthesis as claimed in one of the claims 1 to 6, characterized in that the angle between the shaft axis and the shoulder (12) is about 55°.

8. Prosthesis as claimed in one of the claims 1 to 7, characterized in that the grooves (21) at the medial side (14) of the shaft (10) extend from the distal end thereof only to about 3/4 of the height of the shaft (10) and burls (20) are formed above the grooves (21).

FIG.1

FIG.2

FIG.3

FIG.4

FIG.5

FIG.6

FIG.7

1

FIG.8

FIG.9

FIG.10

FIG.11